(19) Europäisches Patentamt
European Patent Office
Office européen des brevets

(11) Veröffentlichungsnummer: **0 001 246**
**B1**

(12) # EUROPÄISCHE PATENTSCHRIFT

(45) Veröffentlichungstag der Patentschrift: **25.02.81**

(51) Int. Cl.³: **C 07 D 235/18**

(21) Anmeldenummer: **78100852.9**

(22) Anmeldetag: **08.09.78**

(54) **Verfahren zur Herstellung von 2-(3'- bzw. 4'-Aminophenyl)-5(bzw. 6)-aminobenzimidazolen.**

(30) Priorität: **19.09.77 DE 2742093**

(43) Veröffentlichungstag der Anmeldung: **04.04.79 Patentblatt 79/7**

(45) Bekanntmachung des Hinweises auf die Patenterteilung: **25.02.81 Patentblatt 81/8**

(84) Benannte Vertragsstaaten: **CH DE FR GB**

(56) Entgegenhaltungen:
**CA-A-950 146**
**DE-A-2 601 041**

(73) Patentinhaber: **CASSELLA Aktiengesellschaft, Hanauer Landstrasse 526, D-6000 Frankfurt am Main 61 (DE)**

(72) Erfinder: **Bauer, Wolfgang, Dr., Masurenstrasse 4, D-6457 Maintal 3 (DE)**
Erfinder: **Ribka, Joachim, Dr., Rügener Strasse 4, D-6050 Offenbach/Main - Bürgel (DE)**

(74) Vertreter: **Urbach, Hans-Georg, Dr. et al, Hanauer Landstrasse 526, D-6000 Frankfurt am Main 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

EP 0 001 246 B1

Verfahren zur Herstellung von
2-(3'- bzw. 4'-Aminophenyl)-5(bzw. 6)-aminobenzimidazolen

Die vorliegende Erfindung betrifft ein neues Verfahren zur Herstellung von 2-(3'- bzw. 4'-Aminophenyl)-5(bzw. 6)-aminobenzimidazolen der allgemeinen Formel I

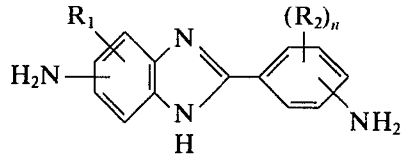

(I)

worin $R_1$ und/oder $R_2$ = Wasserstoff, Alkyl mit 1 bis 4 C-Atomen, Alkoxy mit 1 bis 4 C-Atomen, Chlor oder Brom bedeutet und n für die Zahlen 1 oder 2 steht, durch Kondensation eines 4-Nitro-1,2-diaminobenzols der Formel II

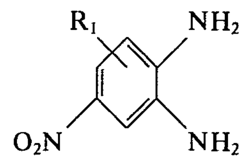

(II)

mit einem 3- bzw. 4-Nitrobenzoesäurechlorid der Formel III

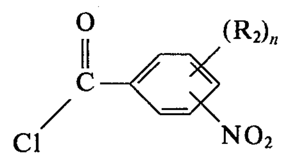

(III)

zu einem 3- bzw. 4-Nitrobenzoesäure-2'-amino-5'-nitroanilid der Formel IV

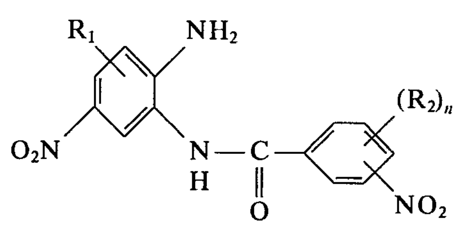

(IV)

und dessen anschließendes Überführen in eine Verbindung der Formel I. Das erfindungsgemäße Verfahren ist dadurch gekennzeichnet, daß die Verbindung der Formel II mit der Verbindung der Formel III bei pH-Werten von 1 bis 10 und Temperaturen von −10° C bis +100° C kondensiert und das entstandene Anilid der Formel IV mit Schwefelwasserstoff, Alkalisulfid, Alkalipolysulfid oder Alkalihydrogensulfid bei pH-Werten von 8 bis 14 und Temperaturen von 50 bis 150° C reduziert und durch Alkalibehandlung bei pH-Werten von 8 bis 14 und Temperaturen von 50 bis 150° C im wäßrigen Medium cyclisiert wird, wobei die Reduktion und die Cyclisierung gleichzeitig in einem Verfahrensschritt oder in beliebiger Reihenfolge in getrennten Verfahrensschritten ausgeführt werden können.

Heterocyclische Diamine der Formel I sind nach verschiedenen literaturbekannten Herstellungsverfahren zugänglich. So ist es aus a) O. Kym, Ber. dtsch. chem. Ges. 32, 2178 (1899); b) DRP 70 862 (26. 7. 1893); Frdl. III, 34 (1894); c) DRP 68 237 (8. 3. 1893); Frdl. III, 711 (1894); d) B. A. Porai-Koshits u. Ch. Frankovskii, Zhur. Obsc. Khim. 28, 928 (1958), e) V. Shchel'tsyn, A. Ya. Kaminskii, T. P. Shapirooskaya, J. L. Valsman, V. F. Adrianov u. S. S. Gitis, Chemistry of Heterocyclic Compounds, USSR, 9, 103 (1973) bekannt, Aminophenylbenzimidazole herzustellen, indem man 3- bzw. 4-Nitrobenzoesäure-2',4'-dinitroanilide zu 3- bzw. 4-Aminobenzoesäure-2',4'-diamino-aniliden reduziert, die durch thermische oder säurekatalysierte Cyclisierung in die heterocyclischen Diamine der Formel I überführt werden. Die Verwendung der als starke Oxidationsmittel wirkenden Trinitroverbindungen als Ausgangsmaterial bei diesem Verfahren bringt große Gefahrenmomente.

Ferner ist das von O. Kym, Ber. dtsch. chem. Ges. 32, 2178 (1899) beschriebene Verfahren, wonach die Trinitroverbindungen in Salzsäure mit Zinn (Methode nach Hinsberg) zu 3- bzw. 4-Aminobenzoesäure-2',4'-diaminoaniliden reduziert werden, welche in Gegenwart von Salzsäure durch Ringschluß in die heterocyclischen Diamine der Formel I übergehen, nachteilig durch die große, heute nicht mehr tolerierbare Abwasserbelastung durch Metallsalze.

Nach den Angaben der Druckschriften

a) J. Pinnow u. F. Wiskott, Ber. dtsch. chem. Ges. 32, 908 (1899);
b) F. F. Stephens u. J. D. Bower, J. chem. Soc. 1949, 2971;
c) B. N. Feitelson, P. Mamalis, R. J. Monalim, V. Petrow, O. Stephenson u. B. Sturgeon, J. chem. Soc. 1952, 2389;
d) N. V. Subba Rao u. C. V. Ratnam, Proc. Indian Acad. Sci. 49 A, 193 (1959)

können 2-(3'- bzw. 4'-Nitrophenyl)-5-(bzw. 6)-nitrobenzimidazole zu den heterocyclischen Diaminen der Formel I reduziert werden.

Dieses Verfahren, wonach heterocyclische Diamine der Formel I aus den entsprechenden heterocyclischen Dinitroverbindungen hergestellt werden können, ist von geringem technischen Wert, da diese Dinitroverbindungen bei der Kondensation von 4-Nitro-1,2-diaminobenzolen II mit 3- bzw. 4-Nitrobenzaldehyden neben den 1-[3'- bzw. 4'-Nitrobenzyl]-2-[3'- bzw. 4'-nitrophenyl]-5-(bzw. 6)-nitrobenzimidazolen entstehen und nur in geringer Ausbeute zugänglich sind.

Dinitrophenyl-benzimidazole können ferner durch Nitrierung von 2-[3'- bzw. 4'-Nitrophenyl]-benzimidazolen oder durch Nitrierung von 2-Phenylbenzimidazol hergestellt werden.

Diese Verfahren sind ebenfalls ohne technische Bedeutung, da 2-[3'- bzw. 4'-Nitrophenyl]-benzimidazole ebenfalls nur schlecht zugänglich sind (vgl. beispielsweise: R. Walter und Th. von Pulawski, J. prakt. Chem. 59, 263 (1899); J. Pinnow und F. Wiskott, Ber. dtsch. chem. Ges. 32, 898 (1899); O. Hinsberg und F. Funcke, Ber. dtsch. chem. Ges. 27, 2187 (1894); V. Sterba, J. Arient und J. Slosar, Collection Czech. Chem. Commun. 31, 1093 (1965) ),und die Nitrierung von 2-Phenyl-benzimidazol zu Gemischen aus 2-Phenyl-5-(bzw. 6)-nitrobenzimidazol, 2-[4'-Nitrophenyl]- und 2-[3'-Nitrophenyl]-5(bzw. 6)-nitrobenzimidazol führt. Die Dinitroverbindungen entstehen dabei in schlechten Ausbeuten (vgl. beispielsweise: D. G. Bapat und M. W. Shirsat, Indian J. Chem. 3, 81 (1965) und V. Sterba, J. Arient und J. Slosar, Collection Czech. Chem. Commun. 31, 1093 (1965) ).

Heterocyclische Diamine der Formel I sind gemäß J. Preston, W. F. DeWinter u. W. L. Hofferberth, Jr., J. Heterocycl, Chem. 1969, 6 (1), 119, durch Kondensation von 1,2,4-Triaminobenzol-di-hydrochloriden mit 3- bzw. 4-Aminobenzoesäuren in Polyphosphorsäure bei 190 – 220° C herstellbar.

Diesem Verfahren haftet der Nachteil an, daß die als Ausgangsverbindungen eingesetzten Triaminobenzole als äußerst autoxidationsempfindliche Verbindungen schwer zu handhaben sind und daß die Diamino-benzimidazole I bei diesem Verfahren nur in geringer Ausbeute isoliert werden können.

Ferner ist aus der DE-OS 26 01 041 ein Verfahren zur Herstellung von 2-(4'-Amino-phenyl)-5-aminobenzimidazol bekannt, bei dem Anilin mit p-Nitrobenzoesäure bei Temperaturen über 170° C zu N-(4'-Nitrobenzoyl)-anilin kondensiert wird, das dann mit Nitriersäure zum N-(4'-Nitrobenzoyl)-2,4-dinitro-anilin nitriert wird. Diese Trinitroverbindung wird isoliert und getrocknet und anschließend mit sulfidischen Reduktionsmitteln im wäßrigen alkalischen Medium reduziert. Dabei entsteht zunächst das $N_1$-(4'-Amino-benzoyl)-1,2,4-triaminobenzol, das isoliert wird und durch Cyclisation im sauren Medium in die gewünschte Verbindung überführt wird. Auch dieses Verfahren besitzt eine Reihe von Nachteilen; so müssen z. B. die als Zwischenprodukte auftretenden Trinitro- und Triaminoverbindungen isoliert werden, von denen die Trinitroverbindung stoßempfindlich ist, so daß zu ihrer Handhabung besondere Vorkehrungen getroffen werden müssen. Ferner gelangt bei der Nitrierung Schwefelsäure ins Abwasser und nach der alkalischen Reduktion muß wieder angesäuert werden. Der sich dabei aus der thiosulfathaltigen Reaktionsmischung abscheidende Schwefel muß abgetrennt werden. Die Endverbindung kann nur über das Hydrochlorid oder nach nochmaligem Alkalischstellen des Ansatzes isoliert werden.

Nach einem weiteren, aus dem kanadischen Patent 9 50 146 bekannten Verfahren zur Herstellung von 2-(4'-Aminophenyl)-5-aminobenzimidazol Ia wird 4-Nitro-1,2-diaminobenzol IIa in einem Lösungsmittelgemisch aus Dimethylacetamid, Triäthylamin und Xylol mit 4-Nitrobenzoylchlorid IIIa zu 4-Nitrobenzoesäure-2'-amino-5'-nitroanilid IVa kondensiert. IVa wird nach Isolierung durch Thermolyse in 2-(4'-Nitrophenyl)-5-nitrobenzimidazol IXa überführt, das ebenfalls isoliert wird und anschließend durch katalytische Hydrierung Ia liefert:

Reaktionsschema

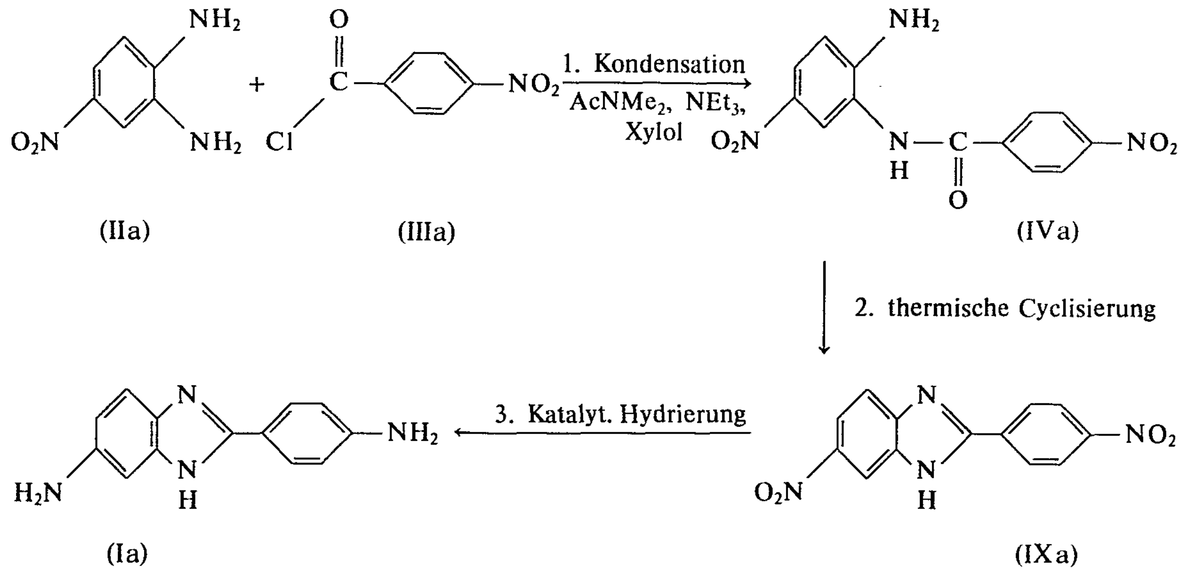


(IIa) (IIIa) (IVa)

(Ia) (IXa)

Das im vorstehenden Reaktionsschema skizzierte bekannte Verfahren hat den Nachteil, daß das heterocyclische Diamin der Formel Ia in getrennten Arbeitsschritten, verbunden mit arbeitsintensiver Isolierung der Zwischenstufen IVa und IXa hergestellt wird, so daß lange Reaktionszeiten, Ausbeuteverluste bei der Zwischenisolierung der Produkte IVa und IXa und der Einsatz einer Vielzahl von Apparaten in Kauf genommen werden müssen. Außerdem bringt das vorstehende Verfahren Schwierigkeiten bei der Durchführung und Aufarbeitung im Hinblick auf die Umwelt mit sich, da bei der Herstellung des Kondensationsprodukts der Formel IVa Dimethylacetamid sowie Triäthylamin als Lösungsmittel eingesetzt werden und die zulässigen MAK-Werte von lediglich 10 ppm für Dimethylacetamid sowie 25 ppm für Triäthylamin nicht überschritten werden dürfen. Hierdurch müssen besondere Maßnahmen bei der Durchführung der Reaktion, der Isolierung des Kondensationsprodukts der Formel IVa und der Regenerierung der Lösungsmittel hinsichtlich der Reinhaltung von Abwasser und Abluft getroffen werden, wodurch weitere erhebliche apparative Aufwendungen erforderlich sind.

Überraschenderweise wurde nun gefunden, daß die Cyclisierung der Anilide der Formel IV sich nicht nur, wie in der Literatur ausschließlich beschrieben, thermisch oder säurekatalysiert, sondern in unvorhersehbar glatter Reaktion auch in alkalischem Medium ausführen läßt. Dies ermöglicht die Durchführung des erfindungsgemäßen Verfahrens zur Herstellung von 2-(3' bzw. 4'-Aminophenyl)-5(bzw. 6)-aminobenzimidazolen der Formel I, das die Nachteile, die den vorstehend genannten literaturbekannten Verfahren anhaften, nicht aufweist.

Die in literaturbekannten Verfahren als starke Oxidationsmittel wirkenden Trinitroverbindungen werden bei dem erfindungsgemäßen Verfahren vermieden. Ferner werden sowohl die Zwischenprodukte der Formel IV als auch die heterocyclischen Diamine der allgemeinen Formel I nach dem erfindungsgemäßen Verfahren in sehr guter Qualität und in ausgezeichneten, gegenüber bekannten Verfahren erheblich verbesserten Ausbeuten erhalten.

Ein besonderer technischer Fortschritt des erfindungsgemäßen Verfahrens gegenüber den bisher bekannten Verfahren ist neben der hohen Ausbeute und sehr guten Qualität der Endprodukte dadurch gegeben, daß es sich wegen der im alkalischen Medium durchgeführten Cyclisierung als einfaches Eintopfverfahren, d. h. ohne Zwischenisolierung und Reinigung auftretender Zwischenstufen durchführen läßt und daß von Schwermetallsalzen freie Fabrikationsabwasser entstehen, deren biologischer und chemischer Sauerstoffbedarf gering ist.

Ein weiterer besonderer Vorteil des erfindungsgemäßen Verfahrens besteht darin, daß als Lösungsmittel bei den Reduktions- und Cyclisierungsschritten Wasser verwendet werden kann und eine Regeneration organischer Lösungsmittel und Maßnahmen zur Einhaltung der zulässigen MAK-Werte somit entfallen. Ein weiterer Vorteil bei der Verwendung von sulfidischen Reduktionsmitteln, insbesondere von Sulfhydratlauge als Reduktionsmittel, ist durch die einfache Lösung von Abwasser- und Abluftproblemen gegeben. Durch gemeinsame Aufbereitung der Abwässer und Abgase aus der Herstellung der Nitrobenzoesäurechloride III und aus dem Reduktions- und Cyclisierungsschritt erhält man ein ökologisch sehr günstiges Abwasser, das keine niedrigwertigen Schwefelverbindungen enthält und dessen biologischer und chemischer Sauerstoffbedarf gering ist.

Das erfindungsgemäße Verfahren wird so durchgeführt, daß man zunächst eine 3- bzw. 4-Nitrobenzoesäure der allgemeinen Formel VI mit überschüssigem Thionylchlorid oder anderen

säurehalogenierenden Verbindungen wie Phosgen, Phosphortrichlorid, Phosphorpentachlorid, Phosphoroxychlorid in an sich bekannter Weise (vgl. Houben-Weyl, Methoden der Organischen Chemie, 4. Auflage, Band VIII, Seite 467 [1952]; DRP 10 26 750) in die entsprechenden 3- bzw. 4-Nitrobenzoesäurechloride der allgemeinen Formel III überführt:

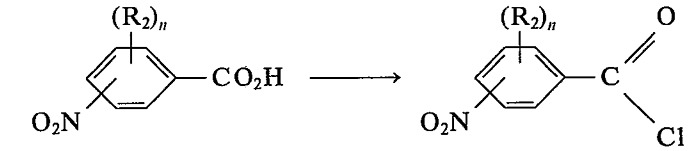

(VI) (III)

Diese Reaktion wird im allgemeinen in Gegenwart geringer Mengen einer organischen Stickstoffverbindung als Katalysator, vorzugsweise Dimethylformamid oder Pyridin, und bei ansteigender Temperatur, vorzugsweise von 20 bis 100° C, durchgeführt. Die Reaktion der 3- bzw. 4-Nitrobenzoesäure VI zum entsprechenden 3- bzw. 4-Nitrobenzoesäurechlorid III wird vorzugsweise ohne Lösungsmittel durchgeführt, sie kann jedoch auch in inerten organischen Lösungsmitteln, beispielsweise Methylenchlorid, Chloroform, Tetrachlorkohlenstoff, Dichloräthan, Trichloräthan, n-Hexan, Cyclohexan, Toluol, Xylol, Monochlorbenzol, o-Dichlorbenzol erfolgen.

Die so erhaltene Schmelze oder Lösung des 3- bzw. 4-Nitrobenzoesäurechlorids III wird — vorzugsweise ohne weitere Reinigung — mit einer wäßrigen Suspension eines 4-Nitro-1,2-diaminobenzols der allgemeinen Formel II zur Reaktion gebracht, wobei 3- bzw. 4-Nitrobenzoesäure-2'-amino-5'-nitroanilide der Formel IV erhalten werden.

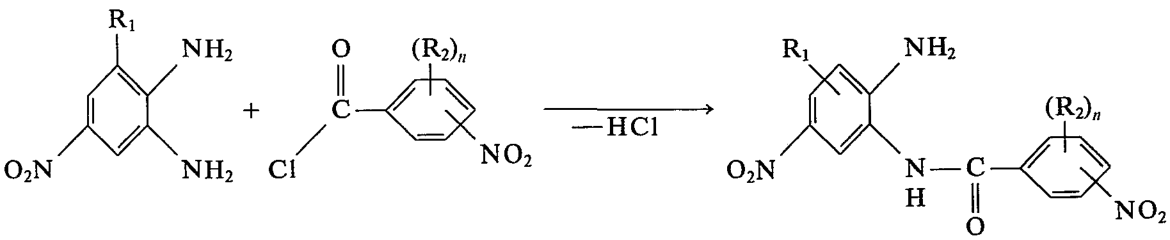

(II) (III) (IV)

Die Kondensationsreaktion wird bei pH-Werten zwischen 1 und 10, vorzugsweise 1 und 5 und bei Temperaturen von −10 bis +100° C, vorzugsweise 0 bis 50° C, durchgeführt.

Zur Einhaltung der bei der Kondensationsreaktion optimalen pH-Werte von 1 bis 10, vorzugsweise 1−5, kann man vor oder während der Reaktion zum Abfangen des freiwerdenden Chlorwasserstoffs geeignete Basen, beispielsweise Natriumhydroxid, Natriumcarbonat, Natriumhydrogencarbonat, Natriumacetat, Trinatriumphosphat, Dinatriumphosphat oder Mono-natriumphosphat, Natriumformiat, Natriumpropionat zufügen. Als bevorzugtes Lösungsmittel bei diesen Kondensationsreaktionen dient zweckmäßigerweise Wasser, um die Regeneration organischer Lösungsmittel zu vermeiden.

Das wäßrige Reaktionsmedium kann jedoch auch mit Wasser mischbare Lösungsmittel, beispielsweise Alkohole, wie Methanol, Äthanol oder Isopropanol oder mit Wasser nicht mischbare Lösungsmittel, beispielsweise Methylenchlorid, Chloroform, Tetrachlorkohlenstoff, Dichloräthan, Trichloräthan, n-Hexan, Cyclohexan, Toluol, Xylol, Monochlorbenzol, o-Dichlorbenzol, enthalten.

Ferner können dem bei den Kondensationsreaktionen verwendeten Reaktionsmedium an sich bekannte Tenside, beispielsweise Aniontenside, Kationtenside, Amphotenside oder nichtionogene Tenside (vgl. Ullmanns Enzyclopädie der technischen Chemie, Band 16, S. 724−748 [1965] oder »Surface Activity« von J. L. Moilliet, B. Collie u. Black, 2. Auflage, Kap. 10−15) zugesetzt werden. Der Zusatz von Tensiden erhöht die Reaktionsgeschwindigkeit der Kondensationsreaktionen. Die Kondensationsreaktionen sind normalerweise nach Reaktionszeiten von 1 bis 6 Stunden bei 0−50° C beendet.

Die Kondensationsprodukte der Formel IV fallen als in Wasser schwerlösliche Verbindungen in Ausbeuten von 80−100% der theoretischen Ausbeute an und können anschließend, vorzugsweise ohne weitere Reinigung und Zwischenisolierung, durch Reduktion und Cyclisierung in die heterocyclischen Diamine der Formel I überführt werden. Nach einer besonders bevorzugten Ausführungsform der vorliegenden Erfindung werden die Reaktionsschritte der Reduktion und Cyclisierung in einem einzigen Verfahrensschritt zusammengefaßt.

Nach dieser bevorzugten Verfahrensvariante (a) wird das Kondensationsprodukt der Formel IV mit einem der oben angeführten sulfidischen Reduktionsmittel, insbesondere mit einem Alkalihydrogensulfid, beispielsweise der technisch üblichen Sulfhydratlauge (Natriumhydrogensulfid), reduziert. Bei diesen Reduktionsverfahren geht das Zwischenprodukt der Formel IV bei pH-Werten zwischen 8 und

14, vorzugsweise 9 und 13, und Temperaturen von 50 bis 150°C, vorzugsweise 80 bis 130°C, überraschenderweise direkt in das heterocyclische Diamin der Formel I über. Das Endprodukt der Formel I wird dabei aus dem wäßrigen Reaktionsmedium in hohen Ausbeuten und ausgezeichneter Qualität isoliert.

Eine andere Verfahrensvariante (b) zur Überführung der Kondensationsprodukte der Formel IV in die heterocyclischen Diamine der Formel I besteht darin, daß man das 3- bzw. 4-Nitrobenzoesäure-2'-amino-5'-nitroanilid IV zunächst mit der zur Reduktion einer Nitrogruppe benötigten Menge eines der oben angeführten sulfidischen Reduktionsmittel, vorzugsweise Natriumhydrogensulfid, selektiv bei pH-Werten von 8 bis 14, vorzugsweise 9 bis 13, bei Temperaturen von 50 bis 150° C, vorzugsweise 80 bis 130° C, zu dem 3- bzw. 4-Aminobenzoesäure-2'-amino-5'-nitroanilid der Formel VII

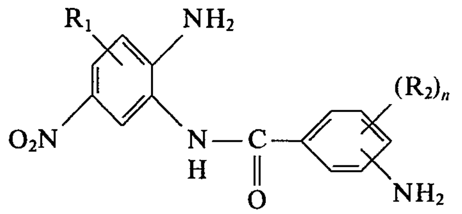

(VII)

reduziert und dieses anschließend bei alkalischen pH-Werten von 8 bis 14, vorzugsweise 9 bis 13, und Temperaturen von 50 bis 150° C, vorzugsweise 80 bis 130° C, zu dem 2-(3'- bzw. 4'-Aminophenyl)-5(bzw. 6)-nitrobenzimidazol der Formel VIII

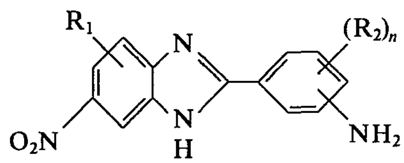

(VIII)

cyclisiert. Die Verbindung der Formel VIII wird anschließend, ebenfalls im alkalischen pH-Bereich von 8 bis 14, vorzugsweise 9 bis 13, und Temperaturen von 50 bis 150°C, vorzugsweise 80 bis 130°C, mit einem der oben angeführten sulfidischen Reduktionsmittel, vorzugsweise Natriumhydrogensulfid, zu dem heterocyclischen Diamin der Formel I weiterreduziert.

Auch nach dieser Verfahrensvariante können die Kondensationsprodukte der Formel IV ohne Isolierung von Zwischenstufen nach einem sogenannten Eintopfverfahren in die Endprodukte der Formel I überführt werden. Hierbei fallen die heterocyclischen Diamine der Formel I ebenfalls in ausgezeichneter Qualität und in hohen Ausbeuten an, was in Anbetracht des über mehrere Stufen ohne Zwischenisolierung und Reinigung der auftretenden Zwischenstufen verlaufenden Prozesses überraschend ist.

Eine weitere Verfahrensvariante (c) besteht darin, daß man das aus 4-Nitro-1,2-diaminobenzol der Formel II und 3- bzw. 4-Nitrobenzoesäurechlorid der Formel III erhaltene Kondensationsprodukt der Formel IV zuerst durch Cyclisierung bei alkalischen pH-Werten von 8 bis 14, vorzugsweise 10 bis 13, und Temperaturen von 50 bis 150°C, vorzugsweise 80 bis 130°C, in das 2-(3'(bzw. 4')-Nitrophenyl)-5(bzw. 6)-nitrobenzimidazol IX

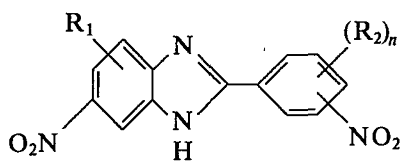

(IX)

überführt, das anschließend, vorzugsweise ohne Zwischenisolierung und Reinigung, im alkalischwäßrigen Reaktionsmedium bei pH 8 bis 14, vorzugsweise 9 bis 13, und Temperaturen von 50 bis 150° C, bevorzugt 80 bis 130°C, durch Reduktion mit einem der oben angeführten sulfidischen Reduktionsmittel, vorzugsweise Natriumhydrogensulfid, zu dem heterocyclischen Diamin der Formel I reduziert wird.

Auch nach Verfahrensvariante (c) erfolgt die Reduktion der zwei Nitrogruppen stufenweise über 2-(3'- bzw. 4'-Aminophenyl)-5(bzw. 6)-nitrobenzimidazole der Formel VIII, deren Zwischenisolierung und Reinigung zur Herstellung der Endprodukte I jedoch nicht erforderlich ist.

Nach dieser Verfahrensvariante lassen sich die Diamine der Formel I ebenfalls in hohen Ausbeuten und in ausgezeichneter Qualität isolieren, was in Anbetracht des mehrstufigen Reaktionsverlaufs überraschend ist und nicht vorherzusehen war.

Die nach dem erfindungsgemäßen Verfahren hergestellten 2-[3'- bzw. 4'-Aminophenyl]-5(bzw. 6)-amino-benzimidazole der allgemeinen Formel I können sowohl in Form der freien Basen, als auch in

Form ihrer Salze mit organischen oder anorganischen Säuren, vorzugsweise ihrer Salze mit Salzsäure oder Schwefelsäure, d. h. als Hydrochloride oder Hydrogensulfate isoliert werden. Sie sind in Form der freien Basen sowie ihrer Hydrochloride oder Hydrogensulfate wertvolle Zwischenprodukte für die Herstellung von Farbstoffen, optischen Aufhellern, temperaturbeständigen Polymeren, beispielsweise Polyamiden, sowie von Pflanzenschutzmitteln.

Als Ausgangsmaterial für die Herstellung von Farbstoffen sind solche Diamine der Formel I bevorzugt, in denen $R_1$ und/oder $R_2$ Wasserstoff, Methyl, Methoxy oder Chlor bedeuten.

Als 4-Nitro-1,2-diaminobenzole der allgemeinen Formel II können bei dem erfindungsgemäßen Verfahren beispielsweise eingesetzt werden:
4-Nitro-1,2-diaminobenzol; 4-Nitro-5-methyl-1,2-diaminobenzol;
4-Nitro-6-methyl-1,2-diaminobenzol; 4-Nitro-6-methoxy-1,2-diaminobenzol;
4-Nitro-6-chlor-1,2-diaminobenzol; 4-Nitro-6-brom-1,2-diaminobenzol;
4-Nitro-5-äthyl-1,2-diaminobenzol; 4-Nitro-5-n-butyl-1,2-diaminobenzol;
4-Nitro-6-äthoxy-1,2-diaminobenzol; 4-Nitro-6-n-butoxy-1,2-diaminobenzol.

Im Hinblick auf die Verwendung der erfindungsgemäß hergestellten Diamine der Formel I zur Herstellung von Azofarbstoffen ist der Einsatz von 4-Nitro-1,2-diaminobenzolen der allgemeinen Formel II, in denen $R_1$ Wasserstoff, Methyl, Methoxy oder Chlor ist, besonders bevorzugt.

Die 4-Nitro-1,2-diaminobenzole II sind durch literaturbekannte Reduktionsverfahren aus den 2,4-Dinitroanilinen zugänglich, beispielsweise durch Reduktion mit Schwefelwasserstoff, Sulfiden und Polysulfiden (vgl. beispielsweise Houben-Weyl, Methoden der Organischen Chemie, Band XI/1, Seite 409—421 [1957], Organic Syntheses, Vol. 21, S. 20 [1941] ), oder durch selektive katalytische Hydrierung (vgl. beispielsweise R. E. Lyle und J. L. La Mattina, Synthesis 1974, 726).

Als 3- bzw. 4-Nitrobenzoesäuren VI, die zur Herstellung der zur Kondensationsreaktion mit den 4-Nitro-1,2-diaminobenzolen II, benötigten 3- bzw. 4-Nitrobenzoesäurechloride III geeignet sind, können beispielsweise eingesetzt werden:
4-Nitrobenzoesäure; 2-Methyl-4-nitrobenzoesäure; 3-Methyl-4-nitrobenzoesäure;
3,5-Dimethyl-4-nitrobenzoesäure; 2-Äthyl-4-nitrobenzoesäure;
2-Methoxy-4-nitrobenzoesäure; 3-Methoxy-4-nitrobenzoesäure;
2,3-Dimethoxy-4-nitrobenzoesäure; 3,5-Dimethoxy-4-nitrobenzoesäure;
2-Methyl-5-methoxy-4-nitrobenzoesäure; 3-Äthoxy-4-nitrobenzoesäure;
3-Methyl-6-methoxy-4-nitrobenzoesäure; 2-Chlor-4-nitrobenzoesäure;
3-Chlor-4-nitrobenzoesäure; 2-Brom-4-nitrobenzoesäure;
3-Brom-4-nitrobenzoesäure; 3-Nitrobenzoesäure; 2-Methyl-3-nitrobenzoesäure;
4-Methyl-3-nitrobenzoesäure; 5-Methyl-3-nitrobenzoesäure;
6-Methyl-3-nitrobenzoesäure; 6-Äthyl-3-nitrobenzoesäure;
2,4-Dimethyl-3-nitrobenzoesäure; 4,6-Dimethyl-3-nitrobenzoesäure;
2-Methoxy-3-nitrobenzoesäure; 4-Methoxy-3-nitrobenzoesäure;
6-Methoxy-3-nitrobenzoesäure; 4-Äthoxy-3-nitrobenzoesäure;
5-Methyl-6-methoxy-3-nitrobenzoesäure; 4-Methoxy-5-methyl-3-nitrobenzoesäure;
4-Methyl-6-methoxy-3-nitrobenzoesäure; 5,6-Dimethoxy-3-nitrobenzoesäure;
2,4-Dimethoxy-3-nitrobenzoesäure; 2,5-Dimethoxy-3-nitrobenzoesäure;
4,5-Dimethoxy-3-nitrobenzoesäure; 2-Chlor-3-nitrobenzoesäure;
4-Chlor-3-nitrobenzoesäure; 5-Chlor-3-nitrobenzoesäure;
6-Chlor-3-nitrobenzoesäure; 2,5-Dichlor-3-nitrobenzoesäure;
2,6-Dichlor-3-nitrobenzoesäure; 4,6-Dichlor-3-nitrobenzoesäure;
2-Brom-3-nitrobenzoesäure; 4-Brom-3-nitrobenzoesäure; 5-Brom-3-nitrobenzoesäure;
6-Brom-3-nitrobenzoesäure; 4,5-Dibrom-3-nitrobenzoesäure; 3-Äthyl-4-nitrobenzoesäure;
3-n-Butyl-4-nitrobenzoesäure; 3-Äthoxy-4-nitrobenzoesäure;
3-n-Butoxy-4-nitrobenzoesäure.

Zur erfindungsgemäßen Herstellung von Diaminen der Formel I, die als Ausgangsmaterial für Farbstoffproduktionen verwendet werden sollen, werden vorzugsweise solche 3- bzw. 4-Nitrobenzoesäurederivate der Formel VI eingesetzt, in denen $R_2$ Wasserstoff, Methyl, Methoxy oder Chlor ist.

Die nachstehenden Beispiele dienen zur Erläuterung des erfindungsgemäßen Verfahrens. Die Teile bedeuten Gewichtsteile, die Temperaturen sind in Celsiusgraden angegeben.

## Beispiel 1

Eine Mischung von 184 Teilen 4-Nitrobenzoesäure, 260 Teilen Toluol und 167 Teilen Thionylchlorid wird mit 3 Teilen Dimethylformamid versetzt und unter Rühren in 7 Stunden bis 80°C erwärmt. Die dabei freigesetzten Gase — Chlorwasserstoff und Schwefeldioxid — werden zweistufig in 200 Teilen Wasser und dann in 400 Teilen 20%iger Natronlauge absorbiert.

Man erhält eine klare Lösung von 4-Nitrobenzoylchlorid in Toluol, die nach Abkühlen auf ca. 20—25°C zu einer Mischung von 153,2 Teilen 4-Nitro-1,2-diaminobenzol und 500 Teilen Eis in 1500 Teilen Wasser gegeben wird. Man läßt ca. 3 Stunden bei 5—10°C nachrühren, heizt die gelbe Suspension des entstandenen 4-Nitrobenzoesäure-2'-amino-5'-nitroanilids (Fp: 348—350°C) auf 50°C und stellt mit einer Lösung von 56 Teilen Natriumhydroxid in 150 Teilen Wasser auf pH 7—7,5.

Anschließend laufen bei 50–60° C 725 g einer 32%igen wäßrigen Sulfhydratlauge (Natrium-hydrogensulfid) zu. Danach heizt man das Reaktionsgemisch auf 95–100° C, wobei eingesetztes Toluol durch Destillation zurückgewonnen wird. Nach einer Reaktionszeit von 10–12 Stunden bei 95–100° C kühlt man auf 5–10° C; das Produkt wird durch Filtration isoliert, mit Wasser gewaschen und bei 70–80° C getrocknet.

Man erhält 206 Teile 2-(4'-Aminophenyl)-5(bzw. 6)-aminobenzimidazol (Fp: 140–142° C) mit einem Reingehalt von 91%.

Das Filtrat der Sulfhydrat-Reduktion wird mit der bei der 4-Nitrobenzoesäurechlorid-Herstellung erhaltenen Natriumbisulfitlösung auf pH 7 gestellt. Dann werden ca. 200 Teile der durch Absorption erhaltenen ca. 20%igen Salzsäure zugefügt. Anschließend wird 1–2 Stunden nachgerührt und vom ausgefallenen Schwefel abfiltriert, wobei ein Filtrat erhalten wird, das Natriumchlorid und Natriumsulfat enthält und frei von niedrigwertigen Schwefelverbindungen ist.

Beispiel 2

Die Kondensation von 153,2 Teilen 4-Nitro-1,2-diaminobenzol mit 204 Teilen 4-Nitrobenzoylchlorid erfolgt nach den Angaben des Beispiels 1. Die Suspension des 4-Nitrobenzoesäure-2'-amino-5'-nitroanilids wird auf 50° C geheizt und mit einer Lösung von 56 Teilen Natriumhydroxid in 150 Teilen Wasser neutralisiert.

Anschließend setzt man bei 50° C 360 g 32%ige wäßrige Sulfhydratlauge zu und erhöht die Temperatur der Reaktionsmischung auf 95–100° C, wobei eingesetztes Toluol durch Destillation zurückgewonnen wird. Die gelbe Suspension des Reduktionsprodukts 4-Aminobenzoesäure-2'-amino-5'-nitroanilid (Fp: 237–239° C) wird dann bei 95° C mit einer Lösung von 20 Teilen Natriumhydroxid in 50 Teilen Wasser versetzt und 1 Stunde bei 95° C und pH 10,5 bis 12 gerührt, wobei Cyclisierung zu 2-(4'-Aminophenyl)-5(bzw. 6)-nitrobenzimidazol (Fp: 285–290° C) eintritt. Die Suspension des Cyclisierungsproduktes wird auf ca. 90° C gekühlt und mit 363 g einer 32%igen Sulfhydratlauge versetzt. Zur Vervollständigung der Reduktion wird 10–12 Stunden bei 95–100° C gerührt, dann auf 5–10° C gekühlt, filtriert und mit Wasser nachgewaschen. Nach Trocknung bei ca. 70–80° C erhält man 207 g 2-(4'-Aminophenyl)-5(bzw. 6)-aminobenzimidazol (Fp: 140–142° C) mit einem Reingehalt von 92%.

Beispiel 3

Die Kondensation von 153,2 Teilen 4-Nitro-1,2-diaminobenzol mit 204 Teilen 4-Nitrobenzoylchlorid erfolgt nach den Angaben des Beispiels 1. Die gelbe Suspension des Kondensationsproduktes 4-Nitrobenzoesäure-2'-amino-5'-nitroanilid wird auf 50° C geheizt und mit einer Lösung von 96 g Natriumhydroxid in 250 Teilen Wasser auf pH 10,5–12 gestellt. Unter Temperaturerhöhung auf 95–100° C wird eingesetztes Toluol durch Destillation zurückgewonnen. Die Cyclisierung zu 2-(4'-Nitrophenyl)-5(bzw. 6)-nitrobenzimidazol (Fp: 350–353° C) ist nach ca. einer Stunde Rühren bei 95–100° C beendet. Die gelbe Suspension des Cyclisierungsprodukts wird anschließend auf ca. 80° C gekühlt und dann mit 725 g einer 31,7%igen wäßrigen Sulfhydratlauge versetzt. Das Reduktionsgemisch wird 10–12 Stunden bei 95–100° C gerührt und dann auf 5–10° C gekühlt. Das Produkt wird durch Filtration isoliert und mit Wasser gewaschen. Man erhält 205 g 2-(4'-Aminophenyl)-5(bzw. 6)-aminobenzimidazol (Fp: 140–142° C) mit einem Reingehalt von 91%.

Der folgenden Tabelle sind weitere Beispiele von 2-(3'-bzw. 4'-Aminophenyl)-5(bzw. 6)-aminobenzimidazolen der Formel I wiedergegeben, die gemäß den vorstehenden Beispielen aus den durch Kondensation von 4-Nitro-1,2-diaminobenzolen der Formel II und 3(bzw. 4)-Nitrobenzoesäurechloriden der Formel III erhaltenen 3(bzw. 4)-Nitrobenzoesäure-2'-amino-5'-nitroaniliden der Formel IV hergestellt werden können.

In der Tabelle ist folgendes angegeben:

In Spalte 1: das eingesetzte 4-Nitro-1,2-diaminobenzol der Formel II
In Spalte 2: das eingesetzte 3(bzw. 4)-Nitrobenzoesäurechlorid der Formel I
In Spalte 3: die Struktur des erhaltenen 2-(3'-bzw. 4'-Aminophenyl)-5(bzw. 6)-aminobenzimidazols der Formel I
In Spalte 4: die Ausbeute an I in % der theoretischen Ausbeute, bezogen auf eingesetztes 4-Nitro-1,2-diaminobenzol II.

| Ausgangsprodukte der Formel (II) | Formel (III) | Endprodukt der Formel (I) | % der Theorie |
|---|---|---|---|
| 4-Nitro-1,2-diaminobenzol | 3-Nitrobenzoylchlorid | 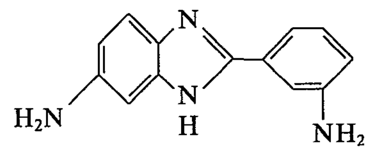 | 77 |
| 4-Nitro-1,2-diaminobenzol | 2-Methyl-4-nitrobenzoyl-chlorid | 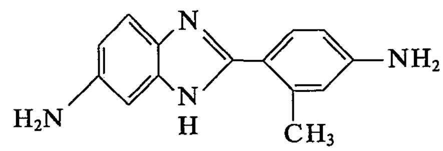 | 79 |
| 4-Nitro-1,2-diaminobenzol | 3-Methyl-4-nitrobenzoyl-chlorid | 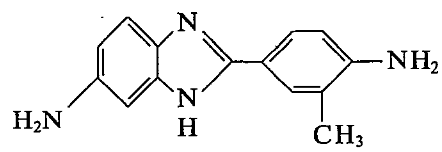 | 81 |
| 4-Nitro-1,2-diaminobenzol | 2-Chlor-4-nitrobenzoyl-chlorid | 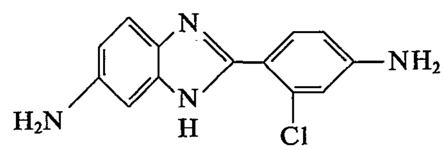 | 69 |
| 4-Nitro-1,2 diaminobenzol | 3-Chlor-4-nitrobenzoyl-chlorid | 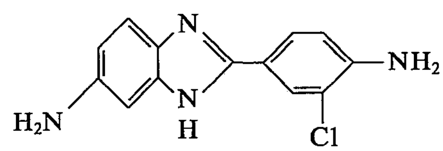 | 80 |
| 4-Nitro-1,2-diaminobenzol | 3-Methoxy-4-nitrobenzoyl-chlorid | 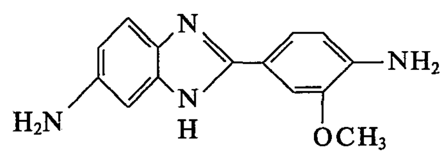 | 81 |
| 4-Nitro-1,2-diaminobenzol | 3-Nitro-4-methoxybenzoyl-chlorid | 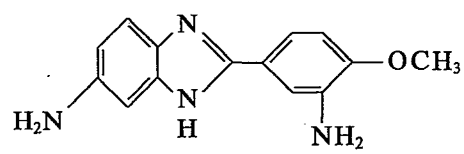 | 79 |
| 4-Nitro-1,2-diaminobenzol | 3-Nitro-4-methylbenzoyl-chlorid | 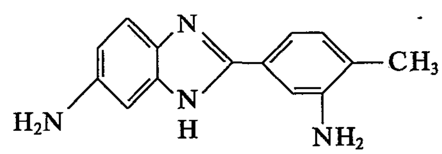 | 76 |
| 4-Nitro-6-chlor-1,2-diaminobenzol | 4-Nitrobenzoylchlorid | 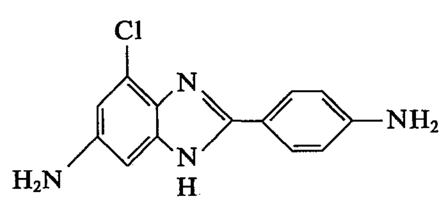 | 82 |
| 4-Nitro-5-methyl-1,2-diaminobenzol | 4-Nitrobenzoylchlorid | 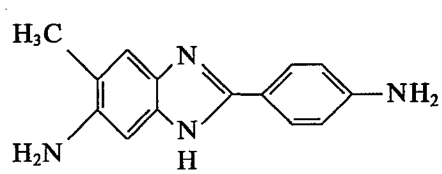 | 81 |

| Ausgangsprodukte der Formel (II) | Formel (III) | Endprodukt der Formel (I) | % der Theorie |
|---|---|---|---|
| 4-Nitro-6-methyl-1,2-diaminobenzol | 4-Nitrobenzoylchlorid | 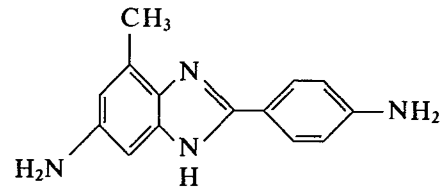 | 80 |
| 4-Nitro-6-chlor-1,2-diaminobenzol | 2-Chlor-4-nitrobenzoyl-chlorid | 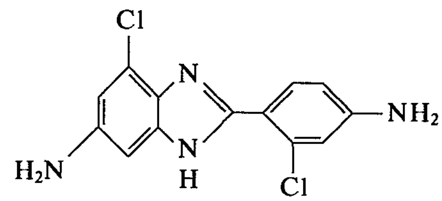 | 75 |
| 4-Nitro-6-chlor-1,2-diaminobenzol | 3-Chlor-4-nitrobenzoyl-chlorid | 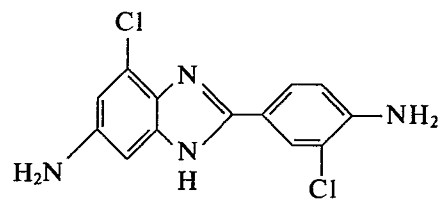 | 81 |

**Patentansprüche**

1. Verfahren zur Herstellung von 2-(3'- bzw. 4'-Aminophenyl)-5(bzw. 6)-aminobenzimidazolen der Formel I

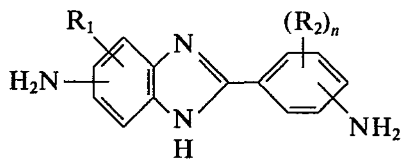

(I)

worin $R_1$ und/oder $R_2$ = Wasserstoff, Alkyl mit 1 bis 4 C-Atomen, Alkoxy mit 1 bis 4 C-Atomen, Chlor oder Brom bedeutet und n für die Zahlen 1 oder 2 steht, durch Kondensation eines 4-Nitro-1,2-diaminobenzols der Formel II

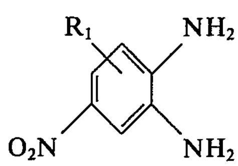

(II)

mit einem 3- bzw. 4-Nitrobenzoesäurechlorid der Formel III

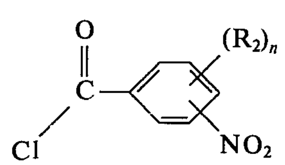

(III)

zu einem 3- bzw. 4-Nitrobenzoesäure-2'-amino-5'-nitroanilid der Formel IV

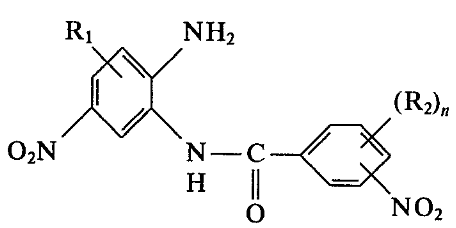

(IV)

und dessen anschließendes Überführen in eine Verbindung der Formel I, dadurch gekennzeichnet, daß die Verbindung der Formel II mit der Verbindung der Formel III bei pH-Werten von 1 bis 10 und Temperaturen von −10°C bis +100°C kondensiert und das entstandene Anilid der Formel IV mit Schwefelwasserstoff, Alkalisulfid, Alkalipolysulfid oder Alkalihydrogensulfid bei pH-Werten von 8 bis 14 und Temperaturen von 50 bis 150°C reduziert und durch Alkalibehandlung bei pH-Werten von 8 bis 14 und Temperaturen von 50 bis 150°C im wäßrigen Medium cyclisiert wird, wobei die Reduktion und die Cyclisierung gleichzeitig in einem Verfahrensschritt oder in beliebiger Reihenfolge in getrennten Verfahrensschritten ausgeführt werden können.

2. Verfahren gemäß Anspruch 1, dadurch gekennzeichnet, daß das Anilid der Formel IV in einem Verfahrensschritt in das 2-(3'- bzw. 4'-Aminophenyl)-5(bzw. 6)-aminobenzimidazol der Formel I überführt wird.

3. Verfahren gemäß Anspruch 1, dadurch gekennzeichnet, daß das Anilid der Formel IV selektiv zum 3(bzw. 4)-Aminobenzoesäure-2'-amino-5'-nitroanilid der Formel VII reduziert wird,

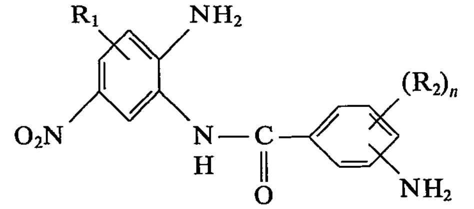

(VII)

das anschließend durch Alkalibehandlung in wäßrigem Medium zu dem 2-(3'- bzw. 4'-Aminophenyl)-5(bzw. 6)-nitrobenzimidazol der Formel VIII cyclisiert wird,

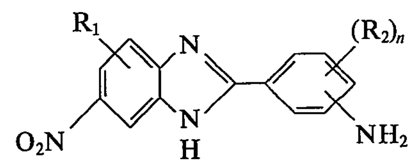

(VIII)

das dann zum 2-(3'- bzw. 4'-Aminophenyl)-5-(bzw. 6)-aminobenzimidazol der Formel I weiterreduziert wird.

4. Verfahren gemäß Anspruch 1, dadurch gekennzeichnet, daß man das Anilid der Formel IV zunächst zum 2-(3'- bzw. 4'-Nitrophenyl)-5(bzw. 6)-nitrobenzimidazol der Formel IX cyclisiert,

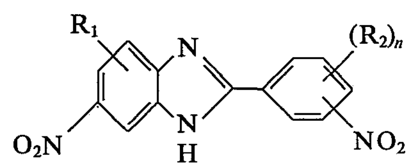

(IX)

das anschließend über die Mononitroverbindung der Formel VIII

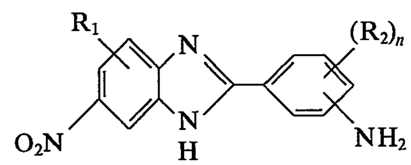

zum 2-(3'- bzw. 4'-Aminophenyl)-5-(bzw. 6)-aminobenzimidazol reduziert wird.

5. Verfahren zur Herstellung von 2-(3'- bzw. 4'-Aminophenyl)-5-(bzw. 6)-aminobenzimidazolen der Formel I nach Anspruch 1 bis 3, dadurch gekennzeichnet, daß das 3- bzw. 4-Nitrobenzoesäurechlorid der Formel III bei der Kondensation mit dem 4-Nitro-1,2-diaminobenzol der Formel II in Form einer Schmelze oder als Lösung in einem inerten organischen Lösungsmittel eingesetzt wird.

6. Verfahren nach den Ansprüchen 1 bis 5, dadurch gekennzeichnet, daß die Reduktion bei pH-Werten von 9 bis 13 durchgeführt wird.

7. Verfahren nach den Ansprüchen 1 bis 6, dadurch gekennzeichnet, daß die Reduktion bei Temperaturen von 80 bis 130°C durchgeführt wird.

8. Verfahren nach den Ansprüchen 1 bis 7, dadurch gekennzeichnet, daß die Cyclisierung durch Alkalibehandlung im wäßrigen Medium bei pH-Werten von 9 bis 13 durchgeführt wird.

9. Verfahren nach den Ansprüchen 1 bis 8, dadurch gekennzeichnet, daß die Cyclisierung durch Alkalibehandlung im wäßrigen Medium bei Temperaturen von 80 bis 130°C durchgeführt wird.

10. Verfahren nach den Ansprüchen 1 bis 9, dadurch gekennzeichnet, daß die Kondensation der Verbindungen II und III bei pH-Werten von 1 bis 5 und Temperaturen von 0 bis 50°C durchgeführt wird.

**Claims**

1. Process for the production of 2-(3′- or 4′-aminophenyl)-5(or 6)-aminobenzimidazoles of the formula I

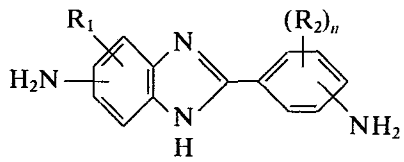

(I)

in which $R_1$ and/or $R_2$ signify hydrogen, alkyl with 1 to 4 carbon atoms, alkoxy with 1 to 4 carbon atoms, chlorine or bromine, and n signifies the numbers 1 or 2, by the condensation of one of the 4-nitro-1,2-diaminobenzenes of the formula II

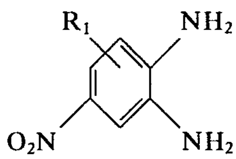

(II)

with a 3- or 4-nitrobenzoic acid chloride of the formula III

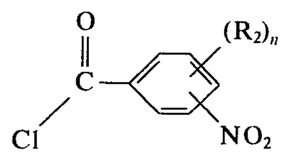

(III)

to form a 3- or 4-nitrobenzoic acid-2′-amino-5′-nitroanilide of the formula IV

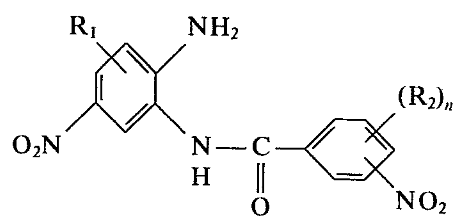

(IV)

and its subsequent conversion into a compound of the formula I, characterised in that the compound of the formula II is condensed with the compound of the formula III at pH values of 1 to 10 and at temperatures between −10°C and +100°C, and that the anilide of the formula IV obtained is reduced with hydrogen sulphide, alkali sulphide, alkali polysulphide oder alkali hydrogen sulphide at pH values of 8 to 14 and at temperatures between 50 and 150°C and is cyclised by alkali treatment in an aqueous medium at pH values of 8 to 14 and at temperatures between 50 and 150°C, it being possible for the reduction and the cyclisation to be carried out simultaneously in a single process step or in separate process steps in any order of sequence.

2. The process of claim 1, characterised in that the anilide of the formula IV is converted in a single process step into the 2-(3′- or 4′-aminophenyl)-5(or 6)-aminobenzimidazole of the formula I.

3. The process of claim 1, characterised in that the anilide of the formula IV is selectively reduced to give the 3-(or 4)-aminobenzoic acid-2′-amino-5′-nitroanilide of the formula VII

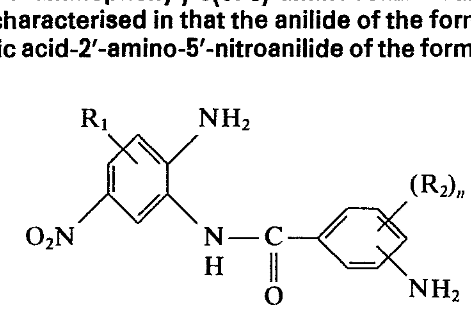

(VII)

which is subsequently cyclised by alkali treatment in an aqueous medium to give the 2-(3′- or

4′-aminophenyl)-5(or 6)-nitrobenzimidazole of the formula VIII

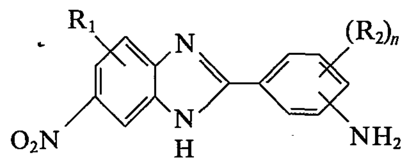

(VIII)

which is then reduced further to give the 2-(3′- or 4′-aminophenyl)-5(or 6)-aminobenzimidazole of the formula I.

4. The process of claim 1, characterised in that the anilide of the formula IV is cyclised to give the 2-(3′- or 4′-nitrophenyl)-5(or 6)-nitrobenzimidazole of the formula IX

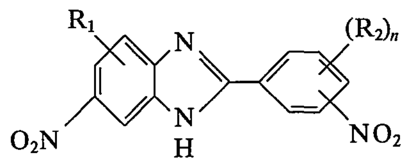

(IX)

which is subsequently reduced via the mononitro compound of the formula VIII

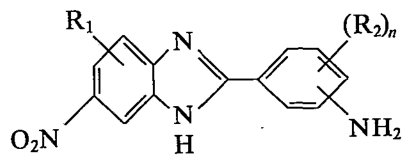

(VIII)

to give the 2-(3′- or 4′-aminophenyl)-5(or 6)-aminobenzimidazole.

5. The process for the production of 2-(3′- or 4′-aminophenyl)-5(or 6)-aminobenzimidazoles of the formula I as claimed in claims 1 to 3, characterised in that in the condensation with the 4-nitro-1,2-diaminobenzene of the formula II the 3- or 4-nitrobenzoic acid chloride of the formula III is employed in the form of a melt or as a solution in an inert organic solvent.

6. The process of claims 1 to 5, characterised in that the reduction is carried out at pH values of 9 to 13.

7. The process of claims 1 to 6, characterised in that the reduction is carried out at temperatures between 80 and 130° C.

8. The process of claims 1 to 7, characterised in that the cyclisation is carried out by alkali treatment in an aqueous medium at pH values of 9 to 13.

9. The process of claims 1 to 8, characterised in that the cyclisation is carried out by alkali treatment in an aqueous medium at temperatures between 80 and 130° C.

10. The process of claims 1 to 9, characterised in that the condensation of the compounds II and III is carried out at pH values of 1 to 5 and at temperatures between 0 and 50° C.

**Revendications**

1. Procédé de préparation des (amino-3′ ou 4′ phényl)-2 amino-5 (ou -6) benzimidazoles répondant à la formule I

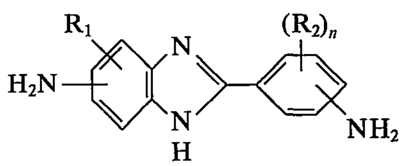

(I)

dans laquelle $R_1$ et/ou $R_2$ représentent l'hydrogène, un alkyle en $C_1-C_4$, un alcoxy en $C_1-C_4$, le chlore ou le brome et n désigne le nombre 1 ou 2, par condensation d'un nitro-4 diamino-1,2 benzène de formule II

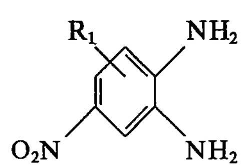

(II)

avec un chlorure nitro-3 ou -4 benzoyle de formule III

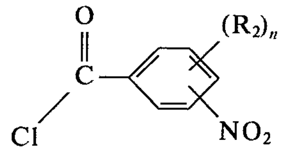

(III)

réaction qui conduit à un amino-2′ nitro-5′ anilide d'acide nitro-3 ou -4 benzoique de formule IV

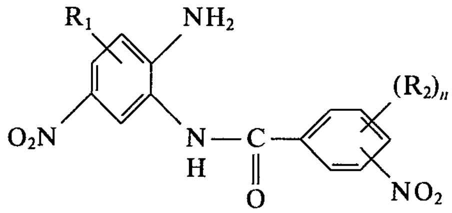

(IV)

puis transformation de celui-ci en en composé de formule I, procédé caractérisé en ce qu'on condense le composé de formule II avec le composé de formule III à des pH de 1 à 10 et à des températures de −10 à +100° C, on réduit l'anilide de formule IV ainsi formé avec le sulfure d'hydrogène ou un sulfure, polysulfure ou hydrogénosulfure de métal alcalin, à des pH de 8 à 14 et à des températures de 50 à 150° C, et on cyclise en milieu aqueux par un traitement alcalin à des pH de 8 à 14 et à des températures de 50 à 150° C, la réduction et la cyclisation pouvant être exécutées en même temps en une seule étape opératoire ou successivement, dans l'ordre que l'on veut, dans des étapes opératoires séparées.

2. Procédé selon la revendication 1, caractérisé en ce qu'on transforme l'anilide de formule IV en 1′(amino-3′ ou 4′phényl)-2 amino-5 (ou -6) benzimidazole de formule I en une seule étape opératoire.

3. Procédé selon la revendication 1, caractérisé en ce qu'on réduit l'anilide de formule IV sélectivement en l'amino-2′ nitro-5′ anilide d'acide amino-3 (ou -4) benzoique de formule VII

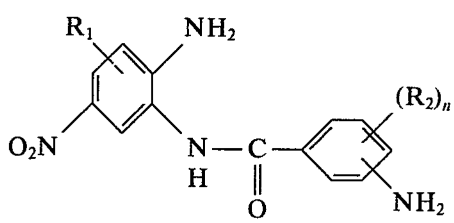

(VII)

puis on cyclise celui-ci par un traitement alcalin en milieu aqueux de manière à le convertir en l'(amino-3′ ou 4′-phényl)-2 nitro-5 (ou -6) benzimidazole de formule VIII

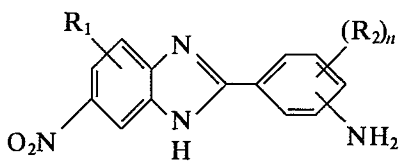

(VIII)

que l'on soumet ensuite à une réduction complémentaire pour obtenir l'(amino-3′ ou 4′-phenyl)-2 amino-5 (ou -6) benzimidazole de formule I.

4. Procédé selon la revendication 1, caractérisé en ce qu'on cyclise d'abord l'anilide de formule IV en (nitro-3′ ou 4′-phényl)-2 nitro-5 (ou -6) benzimidazole de formule IX

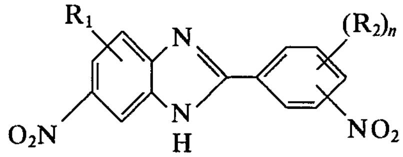

(IX)

que l'on réduit ensuite, en passant par le composé mononitré de formule VIII

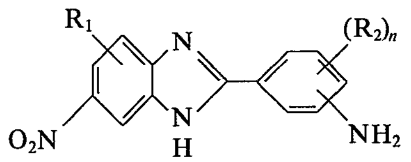

(VIII)

en l'(amino-3′ ou 4′ phényl)-2 amino-5 (ou -6) benzimidazole.

5. Procédé de préparation d'(amino-3′ ou 4′ phényl)-2 amino-5 (ou -6) benzimidazoles de formule I selon l'une quelconque des revendications 1 à 3, caractérisé en ce que le chlorure de nitro-3 ou -4 benzoyle de formule III est mis en jeu, dans la condensation avec le nitro-4 diamino-1,2 benzène de formule II, sous la forme d'une masse fondue ou d'une solution dans un solvant organique inerte.

6. Procédé selon l'une quelconque des revendications 1 à 5, caractérisé en ce qu'on effectue la réduction à des pH de 9 à 13.

7. Procédé selon l'une quelconque des revendications 1 à 6, caractérisé en ce qu'on effectue la réduction à des températures de 80 à 130° C.

8. Procédé selon l'une quelconque des revendications 1 à 7, caractérisé en ce qu'on effectue la cyclisation par un traitement alcalin en milieu aqueux à des pH de 9 à 13.

9. Procédé selon l'une quelconque des revendications 1 à 8, caractérisé en ce qu'on effectue la cyclisation par un traitement alcalin en milieu aqueux à des températures de 80 à 130° C.

10. Procédé selon l'une quelconque des revendications 1 à 9, caractérisé en ce qu'on effectue la condensation des composés (II) et (III) à des pH de 1 à 5 et à des temperatures de 0 à 50° C.